Europäisches Patentamt

European Patent Office

Office européen des brevets

(11) Publication number: **0 072 040**
**B1**

(12) EUROPEAN PATENT SPECIFICATION

(45) Date of publication of patent specification: **13.03.85**

(21) Application number: **82200512.0**

(22) Date of filing: **01.05.82**

(51) Int. Cl.⁴: **C 07 B 57/00,** C 07 C 51/487, C 07 C 59/64

(54) Resolution of d,l 2-(6'methoxy-2'-naphthyl)-propionic acid.

(30) Priority: **18.05.81 IT 2176181**

(43) Date of publication of application:
**16.02.83 Bulletin 83/07**

(45) Publication of the grant of the patent:
**13.03.85 Bulletin 85/11**

(84) Designated Contracting States:
**AT BE CH DE FR GB IT LI LU NL SE**

(56) References cited:
**FR-A-2 035 846**
**US-A-4 209 638**

(73) Proprietor: **BLASCHIM S.p.A.**
**Via Vittor Pisani, 28**
**I-20124 Milano (IT)**

(72) Inventor: **Giordano, Claudio**
**Via Canove Nuove, 23**
**I-36100 Vicenza (IT)**
Inventor: **Villa, Giovanni**
**Via Besozzi, 5**
**I-22068 Monticello Brianza - CO (IT)**
Inventor: **Uggeri, Fulvio**
**Via Grossi, 21**
**I-20073 Codogno - MI (IT)**
Inventor: **Castaldi, Graziano**
**Via Gallina, 5**
**I-28072 Briona - No (IT)**

(74) Representative: **Marchi, Massimo et al**
**c/o CON.P.I. Corso Buenos Aires, 64**
**I-20124 Milano (IT)**

Courier Press, Leamington Spa, England.

## Description

The present invention relates to a new process for preparing the *d* 2-(6'-methoxy-2'-naphthyl)-propionic acid, commonly known as Naproxen, by fractional crystallization in water of a mixture of the corresponding diastereoisomer salts obtained by treatment of a mixture of the d,l 2-(6'-methoxy-2'-naphthyl)-propionic acid with (—) alphaphenylethylamine, separation of the precipitate and subsequent treatment of the precipitate itself with acids, characterized in that the whole process is carried out in water.

The Japanese Patent Application published under the No. 55/055136 discloses a process for preparing Naproxen by resolving a mixture of d,l 2-(6'-methoxy-2'-naphthyl)-propionic acid by using (—)-alpha-phenylethylamine in an organic solvent.

It has now been found that both the salification of d,l-Naproxen with (—)-alpha-phenylethylamine and the subsequent separation of the thus obtained diastereoisomers by fractional crystallization, can be carried out in water.

The amount of (—)-alphaphenylethylamine used ranges from 1 to 0.1 mol for each mol of d,l 2-(6'-methoxy-2'-naphthyl)-propionic acid.

When it is used less than 1 mol of (—)-alphaphenylethylamine the salification of d,l 2-(6'-methoxy-2'-naphthyl)-propionic acid is completed by adding an organic or inorganic base such as an alkylamine, an alkaline hydroxide, ammonium hydroxide.

The salt of (—)-alpha-phenylethylamine and Naproxen which precipitates is separated from the reaction mixture by filtration at a temperature comprised between 0°C and 100°C.

The thus obtained product is treated with acids; the product which precipitates shows a rotatory power comprised between 10° and 66°. When the optical purity of the precipitated product is not high enough the repetition of the above specified operation or subsequent crystallization from water allow to obtain the product having the desired purity.

The yields of each crystallization vary, according to the operation conditions, from 5 to 90%.

The yields of this process can be conveniently improved by adding salts such as alkaline or ammonium salts of carboxylic aliphatic acids.

The following examples are illustrative and representative of the invention.

Example 1

(—)-alpha-phenylethylamine (60 g, 0.5 mols) is added in 5', under stirring, to a suspension of d,l-Naproxen (115 g, 0.5 mols) in water (2 l), warmed at 40°C.

The mixture is heated at reflux under stirring for 30', then it is cooled in 1 hour and 15' at 80°C.

The solid residue is separated by filtration, washed with hot water (80°C) and suspended in diluted hydrochloric acid; the insoluble is filtered, washed with water and dried.

A mixture of the two enantiomers of the 2-(6'-methoxy-2'-naphthyl)-propionic acid having $|\alpha|_D^{20}+49.6°$ is thus obtained.

Example 2

A mixture of (—)-alpha-phenylethylamine (15.1 g; 0.125 mols) and of triethylamine (12.63 g; 0.125 mols) is added in 5', under stirring, to a suspension of d,l-2-(6'-methoxy-2'-naphthyl)-propionic acid (57.5 g; 0.25 mols) in water (1 l), heated at 60°C.

The mixture is kept under stirring, at 60°C for 20 hours. The solid residue is separated by filtration, washed with hot water (60°C; 250 ml) and dried at the air; a mixture of the diastereoisomer salts (28 g) is thus obtained. An aliquot is suspended in diluted hydrochloric acid, the insoluble is filtered, washed with water and dried at the air.

An acid having $|\alpha|_D^{20}+50°$ is thus obtained.

In a parallel test the wet salt is suspended in water (500 ml); the mixture is kept at 60°C for 15 hours and then brought to 90°C and kept at this temperature for 3 hours.

It is then cooled to 60°C in 3 hours and kept at this temperature for 10 hours. The solid residue is separated by filtration, washed with hot water (60°C) and suspended in diluted hydrochloric acid; the insoluble is filtered, washed with water and dried at the air. An acid having $|\alpha|_D^{20}+60°$ is thus obtained.

In a second parallel test carried out in similar conditions, a 10 molar solution of triethylamine acetate (1.58 ml) has been added to the salt suspension in water; and acid having $|\alpha|_D^{20}+62°$ has been thus obtained.

Similar results have been obtained using $NH_4OH$, KOH and NaOH instead of triethylamine.

Example 3

(—)-alpha-phenylethylamine (15.1 g; 0.125 mols) and triethylamine (12.63 g; 0.125 mols) are added under stirring to a suspension of d,l-2-(6'-methoxy-2'-naphthyl)-propionic acid (57.5 g; 0.25 mols) in water (1 l) heated at 100°C.

The mixture is refluxed for 1 hour and then is cooled slowly (10°C per hour) to 25°C and kept at 25°C for 12—14 hours. The solid residue is recovered by filtration and washed with cool water. After drying it is obtained a mixture of the diastereoisomer salts (36 g). An aliquot (4 g) is hydrolyzed with hydrochloric acid to give a product having $|\alpha|_D^{20}+46.87°$.

Example 4

The procedure of Example 3 is repeated by using a 32% of aqueous solution of ammonia (7.5 ml; 0.125 ml) instead of triethylamine.

Yield, 35.4 g. An aliquot of the thus obtained diastomer salts is hydrolyzed to give a product having $|\alpha|_D^{20}+47.22°$.

Another aliquot (10 g) is suspended in water (150 ml) and refluxed for 1 hour; the mixture is then cooled slowly. The thus obtained mixture of diastereoisomer salts (7.9 g) is then hydrolyzed to give a product having $|\alpha|_D^{20}+60.1$.

All the above mentioned rotatory powers have been obtained in chloroform (c=0.01).

## Claims

1. Process for preparing d 2-(6'-methoxy-2'-naphthyl)propionic acid by fractional crystallization of a mixture of the diastereoisomer salts obtained by treatment of the d,l 2-(6'-methoxy-2'-naphthyl)-propionic acid with (—)-alpha phenylethylamine, separation of the precipitate and subsequent treatment of the product with acids characterized in that the whole process is carried out in water.

2. Process according to claim 1, characterized in that from 0.1 to 1 mol of (—)-alpha-phenylethylamine are used for each mol of d,l 2-(6'-methoxy-2-3-naphthyl)-propionic acid.

3. Process according to claim 2, characterized in that when less than one mol of (—)-phenylethylamine is used the formation of the salt of the d,l 2-(6'-methoxy-2'-naphthyl)-propionic acid is completed by adding an organic or inorganic base.

4. Process according to claim 1, characterized in that the precipitation of the salt of the d 2-(6'-methoxy-2'-naphthyl-propionic acid and (—)-phenylethylamine is favoured by adding a salt.

## Revendications

1. Procédé pour la preparation d'acide 2-(6'-méthoxy-2'-naphtyl)-propionique-d par cristallisation fractionnée d'un mélange des sels diastéreo-osimères obtenus par traitement de l'acide 2-(6'-méthoxy-2'-naphtyl)-propionique-d,l par la (—)-alpha-phényléthylamine, séparation du précipité, puis traitement du produit par des acides, caractérisé en ce que tout le procédé est mis en oeuvre dans l'eau.

2. Procédé selon la revendication 1, caractérisé en ce qu'on utilise 0,1 à 1 mol de (—)-alpha-phényléthylamine pour chaque mol d'acide 2-(6'-méthoxy-2-naphtyl)-propionique-d,l.

3. Procédé selon la revendication 2, caractérisé en ce qu'au cas où l'on utilise moins de 1 mol de (—)-alpha-phényléthylamine, la formation du sel d'acide 2-(6'-méthoxy-2'-naphtyl)-propionique-d,l est achevée par addition d'une base organique ou minérale.

4. Procédé selon la revendication 1, caractérisé en ce que la précipitation du sel de l'acide 2-(6'-méthoxy-2'-nephtyl)propionique-d et de la (—)-phényléthylamine est favorisée par l'addition d'un sel.

## Patentansprüche

1. Verfahren zur Herstellung von d-2-(6'-Methoxy-2'-napthyl)-propionsäure durch fraktionierte Kristallisation einer Mischung der diastereoisomeren Salze, die durch Behandeln von d,l-2-(6'-Methoxy-2'-napthyl)-propionsäure mit (—)-α-Phenylethylamin, Abtrennen des Präzipitats und anschließendes Behandeln des Produkts mit Säuren erhalten werden, dadurch gekennzeichnet, daß man das gesamte Verfahren in Wasser durchführt.

2. Verfahren nach Anspruch 1, dadurch gekennzeichnet, daß man 0,1—1 Mol (—)-α-Phenylethylamin pro Mol d,l-2-(6'-Methoxy-2'-napthyl)-propionsäure einsetzt.

3. Verfahren nach Anspruch 2, dadurch gekennzeichnet, daß man, falls man weniger als ein Mol (—)-Phenylethylamin verwendet, d,l-2-(6'-Methoxy-2'-napthyl)-propionsäure, durch Zugabe einer organischen oder anorganischen Base vollständig in das Salz überführt.

4. Verfahren nach Anspruch 1, dadurch gekennzeichnet, daß man die Ausfällung des Salzes von d-2-(6'-Methxy-2'-napthyl)-propionsäure und (—)-Phenylethylamin durch Zugabe eines Salzes fördert.